Europäisches Patentamt

European Patent Office

Office européen des brevets

(19)

(11) Veröffentlichungsnummer: **0 236 468 B1**

(12) # EUROPÄISCHE PATENTSCHRIFT

(45) Veröffentlichungstag der Patentschrift:
17.07.91 Patentblatt 91/29

(51) Int. Cl.$^5$: **A61L 31/00, A61B 17/58**

(21) Anmeldenummer: **86905786.9**

(22) Anmeldetag: **18.09.86**

(86) Internationale Anmeldenummer:
**PCT/EP86/00540**

(87) Internationale Veröffentlichungsnummer:
**WO 87/01595 26.03.87 Gazette 87/07**

(54) ANTIBIOTICAHALTIGES MITTEL UND SEINE VERWENDUNG ALS CHIRURGISCHES KUNSTSTOFFMATERIAL.

(30) Priorität: **19.09.85 DE 3533369**

(43) Veröffentlichungstag der Anmeldung:
**16.09.87 Patentblatt 87/38**

(45) Bekanntmachung des Hinweises auf die
Patenterteilung:
**17.07.91 Patentblatt 91/29**

(84) Benannte Vertragsstaaten:
**AT BE CH DE FR GB IT LI LU NL SE**

(56) Entgegenhaltungen:
**EP-A- 0 003 763**
**EP-A- 0 069 260**
**DE-A- 2 815 934**
**See also references of WO8701595**

(73) Patentinhaber: **BLÖMER, Alois**
**Gildenstrasse 61**
**W-4390 Gladbeck (DE)**

(72) Erfinder: **BLÖMER, Alois**
**Gildenstrasse 61**
**W-4390 Gladbeck (DE)**

(74) Vertreter: **Spalthoff, Adolf, Dipl.-Ing.**
**Pelmanstrasse 31 Postfach 34 02 20**
**W-4300 Essen 1 (DE)**

EP 0 236 468 B1

**Beschreibung**

Die Erfindung betrifft einen Stift zur Einführung in ein in einem Knochen befindliches Bohrloch, welcher aus einem Träger aus Kunststoff auf der Basis von Polymethacrylat oder ähnlichen Materialien besteht, der in gleichermäßiger Verteilung ein Antibiotikum, vorzugsweise Gentamycin, aufweist.

Es ist bekannt, daß Antibiotika, insbesondere Gentamycin, aus Kunststoffen auf Basis von Polymethacrylaten und/oder Polyacrylaten protrahiert freigesetzt werden. Dabei folgt einem steilen initialen Konzentrationsabfall als Ausdruck der Freisetzung aus den äußersten Schichten des Kunststoffs eine fast konstante, über lange Zeiträume langsam abnehmende Freisetzung. Diese antibiotikahaltigen Kunststoffe sind bisher als Knochenzement zum Befestigen von Endoprothesen, z.B. Hüfttotalendoprothesen, oder bei der Auswechselung infizierter Endoprothesen verwendet worden. Zur Infektionsbekämpfung und -prophylaxe werden hierbei die Antibiotika vor der Auspolymerisation des Kunststoffes zugefügt.

Durch die DE-PS 23 20 373 ist ein antibiotikahaltiges Mittel in Kugelform bekannt, welches einen Kusntstoff enthält, wobei das Antibiotikum in gleichförmiger Verteilung im Kunststoff vorliegt. Die Kugeln besitzen einen Durchmesser von 1-20 mm und sind vorzugsweise mit Hilfe von Fäden oder Drähten miteinander verbunden. Dieses Mittel dient beispielsweise zur Ausfüllung von osteomyelitischen Höhlen zur Schaffung eines aseptischen Transplantatlagers usw.

Zur Behandlung von Frakturen ist es bekannt, einen sog. Fixateur externe zu verwenden. Dieser besteht aus Knochennägeln oder -schrauben, welche oberhalb und unterhalb der Fraktur durch die Haut und die Weichteile hindurch bis in die Knochen eingebracht werden. Die äußeren Enden sind mittels Spannelementen miteinander verbunden. Infolge der durch die Anbringung der Knochennägel oder -schrauben entstehenden Weichteildefekte, die für die Dauer der Anlage des Fixateur externe bestehen, ist die Gefahr gegeben, daß Keime in die Weichteile und Knochen vordringen und so zu Entzündungen führen können.

Durch die DE-A-2 815 934 ist ein chirurgischer Stift und dessen Verwendung bekannt, welcher zur Einführung in im Knochen befindliche, infizierte Bohrlöcher dient. Mit diesem chirurgischen Stift soll ein Mittel zur Verfügung gestellt werden, mit dem Infektionen (Bohrlochosteomyelitis) bei der Verwendung chirurgischer Nägel wirksam bekämpft werden können. Zu diesem Zwecke wird vorgeschlagen, daß das Mittel aus einem einen antibakteriellen Wirkstoff enthaltenden Kunststoff besteht und die Form eines Stiftes hat. Der Stift wird in das infizierte Bohrloch eingeschoben, und zwar ist dieses in vielen Fällen sehr leicht ohne Narkose möglich, ebenso wie deren Entfernung.

Der Stift soll den Durchmesser der üblichen chirurgischen Nägel aufweisen, so daß dieser nach Entfernung des Nagels in das infizierte Bohrloch eingeschoben werden kann. Diese bekannte Ausbildung eines chirurgischen Stiftes bringt aber den wesentlichen Nachteil mit sich, daß dessen Verwendung hinter der eigentlichen Therapie herhinkt. In dieser Literaturstelle wird die Lehre gegeben, nach Auftreten von Bohrlochinfektionen die üblicherweise Verwendung findenden chirurgischen Befestigungsmittel, wie Nägel oder Schrauben, zu entfernen, d.h. der Spanner bzw. der Fixateur externe muß abgenommen und die eigentliche primäre medizinische Versorgung unterbrochen werden. In die vorhandenen infizierten Bohrlöcher werden dann die chirurgischen Stifte eingeschoben, um deren Infektionen zu bekämpfen. Nach wirksamer Bekämpfung der Infektionen ist es dann erforderlich, durch erneutes Anlegen des Spanners bzw. des Fixateur externe die eigentliche medizinische Therapie wieder aufzunehmen.

Durch die EP-A-0003763 ist ein Osteosynthesehilfsmittel bekannt, d.h. ein Hilfsmittel für das operative Einrichten von Knochenbrüchen und das Erhalten des dabei erzielten Ergebnisses durch Verschraubung oder Nagelung in Form einer Platte, eines Nagels oder einer Schraube. Das Hilfsmittel soll auf seiner Außenseite mit einer eine konstante Oberflächengestalt aufweisenden, nicht resorbierbaren Trägermasse ausgerüstet sein, in der Antibiotika gleichmäßig verteilt untergebracht sind. Bei dem erörterten Ausführungsbeispiel in Form einer Platte erfolgt die Unterbringung der Trägermasse in offenen Aufnahmeräumen. Über die Art der Anbringung der Trägermasse bei einem Nagel oder einer Schraube sind keine Angaben gemacht, welcher bzw. welche beim operativen Einrichten von Knochenbrüchen in die miteinander zu verbindenden Knochenabschnitte eingebracht werden müssen.

Von diesem Stand der Technik ausgehend liegt der Erfindung die Aufgabe zugrunde, unter Vermeidung vorerwähnter Nachteile einen gattungsgemäßen Stift für chirurgische Zwecke anzugeben, mit dem eine wirksame Bekämpfung von Infektionen bei und nach Anwendung eines sog. Fixateur externe möglich ist.

Erfindungsgemäß wird dies dadurch erreicht, daß der Träger zylinderförmig ausgebildet ist und dessen Innenraum zur Aufnahme eines Knochennagels oder einer Knochenschraube eines Fixateur externe dient. Durch diese Ausbildung kann der Träger den Knochennagel oder die Knochenschraube eines Fixateur externe aufnehmen, so daß mit dem Anlegen des Fixateur externe, also mit dem Einbringen, dessen Schrauben oder Nägel in die Bohrlöcher im Knochen auch gleichzeitig der Träger mit dem Antibiotikum mit in die Bohrlöcher eingebracht wird, so daß mit der medizinischen Versorgung eines Knochenbruches auch gleichzeitig dem Entstehen von Infektionen vorgebeugt wird. Somit bewirken die Träger eine Pophylaxe, so daß es erst gar nicht zu den gefürchteten Bohrlochinfektionen kommt.

Vorteilhaft ist der Träger mit einer Vielzahl von radialen Durchtrittsöffnungen ausgerüstet, welche in dessen Innenraum einmünden.

Der Träger wird über den Knochennagel oder die Knochenschraube eines Fixateur externe geschoben und mit diesem in einen Nagelkanal eingebracht. Das sich bildende Sekret kann über den zwischen äußerer Umfläche des Stiftes und innerer Umfläche des Trägers verbleibenden Kapillarspalt nach außen abgeleitet werden.

Es ist jedoch auch möglich, daß der Träger eine feste Ummantelung für den Knochennagel oder die Knochenschraube eines Fixateur externe bildet, so daß der Träger mit dem Einbringen des Nagelstiftes od.dgl. in den hierfür vorgesehenen Kanal gelangt und während der Anlage des Fixateur externe in dem Kanal verbleibt.

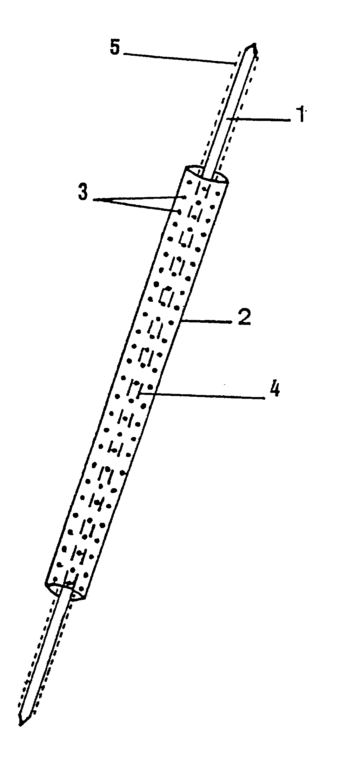

Ein Ausführungsbeispiel der Erfindung ist an Hand der Zeichnung näher erläutert, und zwar zeigt diese in schaubildlicher Darstellung einen Nagel eines Fixateur externe mit einer festen Ummantelung aus dem erfindungsgemäßen Träger.

Mit 1 ist der Nagel eines Fixateur externe bezeichnet, welcher üblicherweise einen Durchmesser von ca. 4 mm besitzt. Der Nagel 1 ist von dem Träger 2 fest ummantelt. Der Träger 2 ist umlaufend und über seine ganze Länge mit einer Vielzahl von radialen Durchtrittsöffnungen 3 versehen, welche in den mittigen Durchtrittskanal 4 des Trägers 2 einmünden. Der Nagel 1 kann in herkömmlicher Weise auch mit Gewinde 5 versehen, also als Schraube ausgebildet sein. Der Träger 2 besitzt einen Durchmesser von ca. 5,6 mm.

Der Träger 2 besteht aus Kunststoff auf der Basis von Polythacrylaten und/oder Polycrylaten oder ähnlichen Materialien. Als Antibiotikum findet vorzugsweise Gentamycin Verwendung.

## Patentansprüche

1. Stift zur Einführung in ein in einem Knochen befindliches Bohrloch, welcher aus einem Träger aus Kunststoff auf der Basis von Polymethacrylat und-/oder Polyacrylat oder ähnlichen Materialien besteht, der in gleichmäßiger Verteilung ein Antibiotikum, vorzugsweise Gentamycin, aufweist, dadurch gekennzeichnet, daß der Träger (2) zylinderförmig ausgebildet ist und dessen Innenraum (4) zur Aufnahme eines Knochennagels oder einer Knochenschraube eines Fixateur externe dient.

2. Stift nach Anspruch 1, dadurch gekennzeichnet, daß der Träger (2) mit einer Vielzahl von radialen Durchtrittsöffnungen (3) ausgerüstet ist, welche in dessen Innenraum (4) einmünden.

3. Stift nach Anspruch 1 oder 2, dadurch gekennzeichnet, daß der Träger (2) auf den Knochennagel oder die Knochenschraube eines Fixateur externe aufgeschoben ist.

4. Stift nach Anspruch 1 oder 2, dadurch gekennzeichnet, daß der Träger (2) eine feste Ummantelung für den Knochennagel oder die Knochenschraube eines Fixateur externe bildet.

## Claims

1. Pin for insertion into a drilled hole in a bone and consisting of a carrier of plastics material based on polymethacrylate and/or polyacrylate or similar materials and comprising a uniformly distributed antibiotic, preferably gentamycin, characterised in that the carrier (2) is cylindrical and the interior (4) thereof is used to accommodate a bone nail or a bone screw of an external fixing device.

2. Pin according to Claim 1, characterised in that the carrier (2) is provided with a plurality of radial openings (3) which lead into the interior (4) thereof.

3. Pin according to Claim 1 or 2, characterised in that the carrier (2) is pushed onto the bone nail or the bone screw of an external fixing device.

4. Pin according to Claim 1 or 2, characterised in that the carrier (2) forms a rigid casing for the bone nail or bone screw of an external fixing device.

## Revendications

1. Broche destinée à être introduite dans un trou réalisé dans un os, qui se compose d'un support en matière plastique à base de polyméthacrylate et/ou de polyacrylate ou de matériaux analogues, et qui présente, réparti de manière uniforme, un antibiotique, de préférence de la gentamicine, caractérisée en ce que le support (2) est cylindrique et en ce que sa chambre intérieure (4) reçoit un clou ou une vis d'un fixateur externe.

2. Broche selon la revendication 1, caractérisée en ce que le support (2) est muni d'une pluralité d'ouvertures de passage (3) radiales qui débouchent dans sa chambre intérieure (4).

3. Broche selon la revendication 1 ou 2, caractérisée en ce que le support (2) est enfilé sur le clou ou sur la vis d'un fixateur externe.

4. Broche selon la revendication 1 ou 2, caractérisée en ce que le support (2) constitue une enveloppe fixe pour le clou ou la vis d'un fixateur externe.

EP 0 236 468 B1

4